Europäisches Patentamt

(19) European Patent Office

Office européen des brevets

(11) Veröffentlichungsnummer: **0 143 413 B1**

(12) **EUROPÄISCHE PATENTSCHRIFT**

(45) Veröffentlichungstag der Patentschrift: 05.06.91

(51) Int. Cl.5 **C07K 3/18**, C07K 17 14, A61K 39/395, G01N 33 94

(21) Anmeldenummer: 84113943.9

(22) Anmeldetag: 17.11.84

(54) **Digitalis-Antikörper, Verfahren zu ihrer Herstellung und ihre Verwendung zur Therapie von Digitalis-Intoxikationen.**

(30) Priorität: 26.11.83 DE 3342870

(43) Veröffentlichungstag der Anmeldung:
05.06.85 Patentblatt 85/23

(45) Bekanntmachung des Hinweises auf die
Patenterteilung:
05.06.91 Patentblatt 91/23

(84) Benannte Vertragsstaaten:
AT BE CH DE FR GB IT LI LU NL SE

(56) Entgegenhaltungen:
US-A- 4 039 652
US-A- 4 434 234

CHEMICAL ABSTRACTS, Band 88, Nr. 11,
März 1978, Seite 39, Zusammenfassung Nr.
69126e, Columbus, Ohio, US; H.R. OCHS et
al.: "Reversal of advanced digitoxin toxicity
and modification of pharmacokinetics by
specific antibodies and Fab fragments", & J.
CLIN. INVEST. 1977, 60(6), 1303-13

CHEMICAL ABSTRACTS, Band 94, Nr. 1, Januar 1981, Seite 282, Zusammenfassung Nr.
2873b, Columbus, Ohio, US; & CA-A-1 085
292 (AMERICAN NATIONAL RED CROSS)
09-09-1980

ALDRICH CHEMIE S.A./N.V.: "Catalogue manuel produits chimiques fins", 1986, Seite
78, Nr. 11339-5, Brüssel, BE

(73) Patentinhaber: BOEHRINGER MANNHEIM
GMBH
Sandhofer Strasse 116
W-6800 Mannheim 31(DE)

(72) Erfinder: Batz, Hans-Georg, Dr. rer. nat.
Traubinger Strasse 63
W-8132 Tutzing(DE)
Erfinder: Jungfer, Herbert, Dr. med.
Beringerweg 10
W-8132 Tutzing(DE)
Erfinder: Lenz, Helmut, Dr. rer. nat.
Waldschmidtstrasse 7
W-8132 Tutzing(DE)
Erfinder: Röder, Albert, Dr. rer. nat.
Bahnhofstrasse 41
W-8124 Seeshaupt(DE)

## Beschreibung

Gegenstand der vorliegenden Erfindung sind Digitalis-Antikörper und ihre Herstellung, die zur Therapie von Digitalis-Intoxikationen verwendet werden können.

Bislang stellte die Therapie schwerer Digitalis-Intoxikationen ein Problem dar. Im Vordergrund stand die Behandlung der Symptome - insbesondere der lebensbedrohenden Herzrythmusstörungen - und eine forcierte Glykosidelimination. Diese Maßnahmen erfordern eine mehrtägige Behandlung auf der Intensivstation. Dennoch ist die Sterblichkeit hoch: ca. 20% der Patienten sterben an nicht beherrschbaren Herzrythmusstörungen. Sehr schwere Vergiftungen kommen in der Praxis vor, wenn entweder die Präparate durch Unachtsamkeit oder infolge eines Suizidversuches zu hoch dosiert werden, oder durch Nierenversagen der Wirkstoff über mehrere Tage nicht ausgeschieden und dadurch in einer toxischen Menge akkumuliert wird.

Es stellte sich deshalb die Aufgabe, ein Mittel zu finden, welches die toxische Wirkung von Glykosiden im Körper neutralisiert und diese rasch und wirkungsvoll aus dem Körper eliminiert.

Diese Aufgabe wird durch die in den Ansprüchen näher beschriebenen Digitalis-Antikörper gelöst, die als Antagonisten nicht nur für Digoxin sondern auch für Digitoxin und eine Reihe anderer Herzglykoside hervorragend geeignet sind.

Bereits Anfang der 6oiger Jahre wurden Digoxin-Antikörper in Radioimmunassays zur Bestimmung von Digoxin eingesetzt (Vgl. DE-OS 23 31 922).

Später publizierte J. Curt et al eine Isolierungsmethode für Schaf-Anti-Digoxin-Fab-Fragmente (im Folgenden Fab genannt) zum therapeutischen Einsatz beim Menschen (Proc. Nat. Acad. Sci. USA 68, 2401, 1971). Zur therapeutischen Anwendung siehe Smith et al, New Engl. J. Med (1982), 1357 - 62 u. (1976), 1125 - 1129. Schafe werden dabei mit an Albumin gebundenem Digoxin immunisiert, daß Serum gewonnen und die Gammaglobulinfraktion mit Ammonsulfat nach an sich bekannter Methode ausgefällt. Die Globuline werden mit Papain in homogener Lösung gespalten und die spezifischen Glykosid-bindenden Fab-Fragmente mit einer Ouabain-Cellulose-Matrix adsorbiert und anschließend mit Ouabain wieder eluiert. Der Ouabain-Fab-Komplex wird durch Denaturierung des Proteins mit Guanidinhydrochlorid dissoziiert und Ouabain und Guanidin durch Dialyse abgetrennt. Durch Dialyse gegen physiologische Pufferlösung wird das durch die Guanidinbehandlung denaturierte Fab-Protein wieder renaturiert. Für eine wirtschaftliche Herstellung von Digoxin-Antikörpern weist dieses Verfahren mehrere Nachteile auf:

1. Durch die Bindung an Ouabain werden bevorzugt solche Antikörper isoliert, die eine starke Kreuzreaktion mit Ouabain aufweisen und Antikörper, die spezifisch für Digoxin sind und deshalb mit Ouabain nicht reagieren, verworfen.

2. Bei der Dissoziation des Ouabain-Fab-Komplexes wird der Protein-Anteil denaturiert, wobei es zwangsläufig auch zu irreversiblen Veränderungen der Proteine kommt. Darüber hinaus erfordert die Dialyse sehr große Volumina, lange Zeit und eine sorgfältige Kontrolle, um einerseit Guanidinhydrochlorid vollständig zu entfernen und andererseits eine möglichst umfangreiche Renaturierung zu bewirken.

3. Als Immunadsorbens wird bei diesem Verfahren Ouabain verwendet, welches über Ribonuclease an Cellulose gekoppelt ist. Als organischer Naturstoff ist Cellulose praktisch immer mit Bakterien kontaminiert, so daß ein keimarmes und pyrogenfreies Arbeiten vor allem in größerem Maßstab und bei wiederholter Verwendung des Adsorbens nicht möglich ist.

Es stellte sich damit die weitere Aufgabe ein wirksames Verfahren zur Herstellung eines für Digoxin- und Digitoxinspezifischen, keim- und pyrogenarmen Antikörpers zu finden.

Diese Aufgabe wird dadurch gelöst, daß man die in der oben beschriebenen Weise erhaltene Gammaglobulinfraktion an einem für die Digoxinantikörper spezifischen Adsorbens, welches aus einem anorganischen Träger besteht, an den Digitoxin kovalent gebunden ist, adsorbiert und die mit Digitoxin nicht bindenden Anteile mit einem geeigneten Puffer auswäscht. In einem zweiten Schritt wird das mit Digoxin-spezifischen IgG beladene Adsorbens mit Papain behandelt und die Fab-Anteile des Antikörpers vom Fc-Teil abgespalten. Papain und freigesetzte Fc-Teile können eluiert und anschließend die Digoxin-spezifischen Fab-Fragmente mit einem stärkeren Elutionsmittel von der Säule gelöst und gewonnen werden.

Die so gewonnenen Fab-Fragmente können in an sich bekannter Weise durch Gelpermeationschromatographie, Ionenaustauscherchromatographie oder fraktionierte Fällung sowie durch Ultrafiltration weiter gereinigt werden, um auch letzte Spuren von störenden Verunreinigungen zu entfernen.

Als anorganische Trägermatrix wird vorzugsweise ein Porenglas mit geeigneter Porengröße verwendet, jedoch sind auch andere anorganische Träger mit genügend hoher Oberfläche (ca. 1 - 20 $m^2/g$, vorzugsweise 5 - 15 $m^2/g$) und entsprechenden Eigenschaften wie Kieselgel, Aluminiumoxidgel oder andere brauchbar. Um die Oberfläche des Trägers für die Bindung des Digitoxins zu präparieren, wird das Glas zum Beispiel mit einem Spacer, wie Trialkoxysilylpropylamin oder einer anderen Verbindung die freie Aminogruppen oder andere mit Digitoxin reagierende Gruppen erzeugt, behandelt. Der so erhaltene Träger wird mit Digitoxin, welches mit Perjodat in an sich bekannter Weise zum so genannten "Digitoxindialdehyd" oxidiert ist, umgesetzt (für die Oxidation vgl. Sardo et al, Chem. Pharm. Bul. 18, 94 - 99 (1970) ) und die erhaltene "Schiffsche Base" reduziert.

Das so erhaltene Adsorbens hat folgende wesentliche Vorteile gegenüber dem von Curt et al. publizierten (siehe oben):

1. Durch die Verwendung eines anorganischen Trägers sind diese Adsorbentien gegen Bakterienbefall praktisch inert und mechanisch sehr stabil, so daß sie wiederholt verwendet werden können.

2. Dadurch daß Digitoxin kovalent über eine durch Papain nicht spaltbare Brücke an das Glas gebunden ist, kann die Fragmentierung der Antikörper direkt an dem Adsorbens erfolgen. Es ist dadurch möglich, nicht nur das Fremdeiweiß sondern auch die bei der Papainspaltung auftretenden Fc-Anteile sowie das Papain selbst in schonender Weise von der Säule zu waschen und dadurch von den adsorbierten Fab-Körpern zu trennen. Durch diese Fragmentierung wird andererseits die Bindungsfähigkeit des Adsorbens für die zurückbleibenden Fab-Fragmente so geschwächt, daß diese ohne Denaturierung mit einem geeignetem Desorptionsmittel wieder von der Säule abgelöst werden können.

3. Durch die Verwendung von digitoxinhaltigen Adsorbentien, werden diejenigen Antikörper besonders fest gebunden, die für Digitoxin, Digoxin und ihre Derivate spezifisch sind, so daß damit letztlich Fab-Körper gewonnen werden, die als Antagonisten für die vorerwähnten Glykoside besonders wirksam sind.

Die Adsorption der Digoxin-Antikörper aus dem IgG-haltigen-Serum an die Digitoxin-haltigen Adsorbentien, erfolgt in den für solche Trennungen üblichen neutralen bis schwach sauren (pH 6-7,5) wässrigen Puffer- und Salzlösungen. Neutrale Phosphatpuffer in Konzentrationen von 0,01 - 0,5 M, Natriumchlorid und Ammoniumsulfat als Salze in Konzentrationen von 0,01 - 0,5 M, insbesondere 0,025 - 0,05, werden bevorzugt verwendet. Zusätzlich kann die Lösung noch 0,01 - 0,1 Gew.% eines üblichen Netzmittels enthalten. Mit diesen Lösungen können auch nach der Papain-Spaltung die Fc-Anteile sowie das Papain aus der Säule ausgewaschen werden.

Stärker saure Puffer in höherer Konzentration führen zu einer Ablösung der Antikörper bzw. Fab-Fragmente, gleichzeitig jedoch auch zu einer teilweisen Denaturierung. Bevorzugt wird die Ablösung der Fab-Fragmente deshalb mit verdünnter Mineralsäure, insbesondere Salzsäure in Konzentrationen von 1 - 10 mM durchgeführt. Konzentrationen von 2 - 5 mM werden bevorzugt, da sie eine rasche Ablösung mit minimaler Denaturierung der Fab-Körper verbinden und sich durch Dialyse leicht von diesen abtrennen lassen.

In dem folgenden Beispiel wird das erfindungsgemäße Verfahren näher ausgeführt, ohne daß dadurch eine Einschränkung erfolgen soll

Beispiel

1. Herstellung von Schaf-Anti-Digoxin-Serum

Immunogen

Digoxin-glutaryl-O-hydroxysuccinimid wird in wässriger Lösung mit Protein, bevorzugt Rinderalbumin oder Edestin (einem Protein aus Hanfsaat) umgesetzt. Nach Abtrennung des Überschusses an Digoxinderivat sind mehrere Digoxinmoleküle pro Molekül Protein an der entständigen Digitoxose über eine Glutarylkette an das Protein gekuppelt.

Immunisierung

Ca. 0,5 mg Digoxin-Immunogen werden in komplettem Freund's Adjuvans intradermal in gesunde Schafe von mindestens 45 kg Körpergewicht appliziert. In Abständen von 2, später 4 Wochen wird das Immunogen in gleicher Aufbereitung intramuskulär und subkutan zur Steigerung der Immunantwort erneut injiziert. Nach 2 bis 4 Monaten erreicht der Titer im Serum der Tiere 1 mg Digoxinspezifisches IgG ml.

Serumabnahmen, Analytik, Poolbildung

Gesunde Tiere, die in einer Probeabnahme den genannten Titer erreicht haben, können über mindestens ein Jahr wöchentlich zur Blutabnahme aus der Halsvene herangezogen werden. In Stichproben der Abnahme wird laufend der Titer mit einem Radioimmuntest oder Enzymimmuntest kontrolliert. Bindungskonstanten wurden ebenfalls in Stichproben durch Scatchard-Plot Auswertung der Bindung von radioaktiv markiertem Digoxin durch definierte Verdünnungen der Antiserumproben bestimmt. Es ergaben sich in Übereinstimmung mit der Literatur Werte von $5 \times 10^9$ bis $5 \times 10^{10}$ (Liter Mol). Um Chargenschwankungen des in die Fab-Isolierung einzusetzenden Antiserums möglichst gering zu halten, werden Gebinde von 20 - 50 l aus Einzelabnahmen von mehreren Schafen über einen längeren Zeitraum gebildet.

2. Herstellung des Immunadsorbens

Amingruppenhaltiges Glas

940 g Controlled Pore Glass (Electro Nucleonics, Inc., Fairfield, USA) wird zur Entfernung von Kleinpartikeln und Verunreinigungen in wässriger Suspension mit Ultraschall behandelt, dann mit 65%iger Salpetersäure bei 80 - 90°C drei Stunden erhitzt. Nach Auswaschen der Säure wird das Glas bei 150°C getrocknet. Das gereinigte Glas wird in 2,7 l wasserfreiem Dimethylsufoxid mit 300 ml 3-(Triäthoxysilyl)-propylamin ca. 20 Stunden bei 85°C am Rückflußkühler langsam gerührt. Das überschüssige Silylamin wird mit Isopropanol ausgewaschen und das aminierte Glas erneut getrocknet (60°C, schwaches Vakuum). Ausbeute: ca. 1,8 l Aminopropyl-Glas (750 g).

Oxidation von Digitoxin

6 g Digitoxin werden in 450 ml einer 2 : 1 Ethanol/Wassermischung mit der stöchiometrisch gleichen Menge Natriumperjodat (1,7 g) 20 Std. bei Raumtemperatur umgesetzt. Unlösliche Ausfällung wird abfiltriert und verworfen. Der Überstand wird am Rotationsverdampfer zum Trocknen gebracht. Der Rückstand wird mit 2 x 100 ml Wasser gewaschen, dann am Vakuum über Calciumchlorid getrocknet. Ausbeute 4 - 5 g oxidiertes Digitoxin.

Digitoxin-Glas-Adsorbens

4,5 g oxidiertes Digitoxin werden in 2,25 l eines 2 : 1 Ethanol-Wassergemisches gelöst, mit 750 g Aminopropyl-Glas versetzt und unter langsamem Rühren 20 Std. bei Raumtemperatur umgesetzt. Das Glas wird durch Filtration abgetrennt. Im Durchlauf wird Digitoxin durch Extinktionsmessung im UV (260 nm) bestimmt und daraus die Menge fixiertes Digitoxin errechnet. (Sollwert: 3 mg Digoxin/ml Glasvolumen). 4 g Natriumborhydrid werden in 1 l bidest. Wasser gelöst, mit 2 l Äthanol verdünnt und auf das Digitoxin derivatisierte Glas gegeben. Unter mehrmaligem Umschütteln wird über 2 Std. bei Raumtemperatur der pH durch Zusätze von 2 N Salzsäure auf Werte von 7,0 bis 7,4 eingestellt. Dabei wird das zunächst über labile Schiffsbasenbindung an das Glas fixierte Digitoxin in eine beständige Fixierung überführt. Das Digitoxin-Glas-Immunadsorbens wird gründlich mit Ethanol/Wasser/Ethanol gewaschen, dann bei schwachem Vakuum getrocknet. Ausbeute ca. 1,8 l Immunadsorbens.

Vorbereitung des Immunadsorbens für den unmittelbaren Gebrauch

Das Adsorbens wird mit Puffer A (50 mM Phosphat pH 7,0/0,15 M NaCl/0,1 % Natriumazid) aufgeschlämmt, in eine Glassäule mit Fritte gefüllt und mit einer Lösung von 0,5 M NaCl/0,05 % Tween 20, dann bidest. Wasser, dann 1 M Propionsäure gewaschen. Zuletzt wird mit Puffer A äquilibriert. In der Fab-Aufreinigung gebrauchtes Immunadsorbens wird durch Waschen mit 1 M Propionsäure regeneriert und im Puffer A bei 4°C gelagert. Das Adsorbens ist mehrmals ohne Beeinträchtigung der Eigenschaften einsetzbar. Direkt vor Gebrauch wird das frische Adsorbens vorgewaschen.

3. Herstellverfahren für Schaf-Anti-Digoxin-Fab

Die zur Aufreinigung verwendeten Lösungen werden im Autoklaven oder durch Membranfiltration sterilisiert. Das zur Herstellung der Lösungen verwendete Wasser ist durch Destillation pyrogenfrei. Gefäße und Geräte sowie andere Hilfsmaterialien (z.B. Chromatographiematerialien) werden je nach Verträglichkeit durch Erhitzung oder durch Behandlung mit 0,5 M NaOH pyrogenfrei gemacht.

Vorreinigung von Schaf-Anti-Digoxin-Serum

30 l Antiserum werden zur Entfernung von Lipoproteinen mit 300 g Aerosil 1 Std. bei Raumtemperatur gerührt. Nach Abtrennung des Aerosils durch Zentrifugation werden aus dem auf 4°C abgekühlten Überstand durch Zusatz von festem Ammonsulfat bis zur Konzentration von 1,8 M die Gamma-Globuline gefällt. Durch Zentrifugation wird der Niederschlag gesammelt, in einer Lösung von 0,15 M Natriumchlorid/0,1% Azid gelöst und gegen Puffer (15 mM Phosphat pH 7,1 50 mmol NaCl/0,002 % Hibitane) bei 4°C dialysiert. Das Dialysat wird auf eine Diethylamincellulosesäule (De 52-Cellulose, Whatman, Maidstone, England), mit dem Austauschervolumen von 16,2 l aufgetragen und mit Puffer der gleichen Zusammensetzung wie der Dialysepuffer eluiert. Das mit diesem Puffer von der Säule ablaufende Protein ist die für die Fab-Herstellung dienende IgG-Fraktion aus Schaf-Anti-Digoxin-Serum. Proteinausbeute ca. 500 - 700 g. Gehalt an digoxinspezifischen Antikörpern ca. 85 - 90 % des Anfangstiters im Antiserum.

Spezifische Adsorption

Ca. 250 g IgG-Fraktion in ca. 10 l Lösung mit einem Gehalt an digoxinspezifischem IgG von 15 g wird auf eine Pufferkonzentration von 50 mM Phosphat/0,15 M NaCl/0,002 % Hibitane/pH 7,0 (Puffer B) gebracht. Diese Lösung wird bei Raumtemperatur im Verlauf von ca. 2 Stunden über eine Säule, gefüllt mit 2,4 l Digitoxin-Glas-Immunadsorbens, gepumpt. Die spezifischen IgG Anteile binden zu 90 % an das Adsorbens. Das Adsorbens wird mit Puffer B, dem 0.35 M NaCl und 0,05 % Tween 20 zugesetzt sind, gewaschen, dann mit einem Puffer C (75 mM Phosphat pH 7,0/0,15 M NaCl/2 mM Ethylendiamintetraessigsäure/10 mM Cystein·0,01 % Hibitane), der für die anschließende Papainspaltung geeignet ist, äquilibriert.

Enzymatische Fragmentierung

Das mit Digoxin-spezifischem IgG beladene Adsorbens wird ohne Verzögerung aus der Säule in einen Glasstutzen überführt und mit 240 mg Papain, gelöst in 2 l Puffer C, versetzt. Bei langsamem Rühren mit einem Metallflügelrührer wird 4 Stunden 15 Minuten bei 37°C inkubiert. Durch Messung von Proben des Überstandes der Adsorbenssuspension im UV-Fotometer (280 nm) zu verschiedenen Zeitpunkten während der Inkubation kann die Kinetik der Freisetzung von Fc-Protein und damit die Fab-Bildung verfolgt werden. Am Ende der Inkubation wird das Adsorbens wieder in eine Säule gefüllt und mit Puffer B gewaschen. Zur Absättigung von freien SH-Gruppen aus der Papainspaltung in Gegenwart von Cystein wird das Adsorbens mit Puffer (75 mM Phosphat pH 7,5/0,15 M NaCl/0,01 % Hibitane) gespült, dem 10 mM Jodacetamid zugesetzt sind. Der Jodacetamidpuffer bleibt 2 Stunden bei Raumtemperatur in Kontakt mit dem Adsorbens. Dann wird mit einer Lösung von 0,15 M NaCl/0,01 % Hibitane überschüssiges Jodacetamid ausgewaschen und mit 30 mM NaCl-Lösung das Adsorbens für die Elution vorbereitet.

Elution (RT)

Zur Elution von spezifischem Fab wird 3 mM wässrige Salzsäure über die Adsorbenssäule gepumpt. Das Fab-Protein wird im Ablauf der Säule durch Messung der UV-Adsorption (280 nm) aufgesucht und gesammelt. 5 - 7 l Lösung, enthaltend ca. 11 g Protein, werden durch Ultrafiltration auf ca. 200 - 250 ml konzentriert, dann sofort lyophilisiert.

Gelpermeationschromatographie (4°C)

Eine Säule von 1 m Länge wird mit 7,5 l Sephacryl S 200 (Pharmacia, Schweden) gefüllt und mit Puffer (5 mM Phosphat pH 7,0/0,5 M NaCl/0,002 %Hibitane äquilibriert. Ca. 5 g Fab Lyophilisat werden in 110 ml Äquilibrierpuffer gelöst und über die Gelsäure chromatographiert. Durch Aufzeichnung der UV-Absorption im Eluat wird der Fab Peak mit Molukargewicht 50.000 festgestellt und gesammelt. Ausbeute ca. 3,75 g gelchromatographisch einheitliches Protein. Reste von IgG, höhermolekularen Fab-Aggregaten und kleineren Bruchstücken sind abgetrennt. Die Fab-Lösung von ca. 700 - 800 ml wird durch Ultrafiltration auf ca.

150 ml konzentriert, dann dialysiert gegen Puffer (15 mM Phosphat pH 7,5·0,15 M NaCl).
Die Chromatographie wird für den 2. Anteil des Fab-Lyophilisats an der gleichen Säule wiederholt. Beide chromatographierten Anteile werden für die weiteren Schritte wieder vereinigt. Zwischenlagerung des ersten Anteils - 20° tiefgefroren.

Passage durch DEAE-Ionenaustauscher (4°C)

400 ml De 52-Cellulose (vorbehandelt mit 0.5 N HCl und 0,5 M NaOH zur Sterilisierung und Pyrogenbefreiung) werden in eine Säule gefüllt und mit 10 mM Phosphatpuffer pH 7,1 äquilibriert. Fab-Protein aus der vorstehenden Stufe wird durch Ultrafiltration auf eine Proteinkonzentration von ca. 50 mg.ml eingestellt, dann die Proteinlösung über die De 52 Säule passiert. Das Fab-Protein läuft fast unverzögert durch, Spuren von Fremdprotein (z.B. Albumin, Fc usw.) werden festgehalten. Das Fab-Protein im Ablauf wird gesammelt, im Bedarfsfall durch Ultrafiltration konzentriert auf ca. 15 - 20 mg Protein ml, an der Elektrode der pH-Wert ad 7,0 - 7,1 korrigiert und durch 0,2 μ Membranfilter steril filtriert. Schrittausbeute 95 %. Endausbeute 5 - 6 g Schaf-Anti-Digoxin-Fab.
Das sterilfiltrierte Endprodukt wird keimarm in Glasflaschen abgefüllt und bei -20° tiefgefroren gelagert.

**Ansprüche**

1. Verfahren zur Herstellung von Digitalisantikörpern, wobei geeignete Säugetiere mit einem an Protein gebundenen Digoxin immunisiert werden, das Serum der Tiere gewonnen wird, die immunglobulinhaltige Eiweißfraktion in an sich bekannter Weise separiert wird, die immunologisch aktiven Globuline an einer immunologisch aktiven Säule adsorbiert und von den übrigen Proteinen getrennt werden, die Antikörper wieder von der Säule eluiert, die Fab-Fraktionen mit Papain abgespalten und gereinigt werden, dadurch gekennzeichnet, daß man als immunologisch aktives Adsorbens eine anorganische Matrix großer Oberfläche verwendet, an die über mit Papain nicht spaltbare Spacer kovalent Digitoxinaldehyd gebunden ist und die Abspaltung der Fab-Fraktion aus dem Antikörper auf der Matrix vornimmt.

2. Verfahren gemäß Anspruch 1. dadurch gekennzeichnet, daß als anorganisches Trägermaterial poröses Glas verwendet wird.

3. Verfahren nach einem der Ansprüche 1 oder 2, dadurch gekennzeichnet, daß als Spacer Triethoxysilylpropylamin verwendet wird.

4. Verfahren nach einem der Ansprüche 1 bis 3, dadurch gekennzeichnet, daß durch Oxidation von Digitoxin mit Perjodat erhaltener Digitoxinaldehyd an die Matrix als Schiff'sche Base gebunden und mit Natriumborhydrid die Schiff'sche Base reduziert wird.

5. Verfahren nach einem der Ansprüche 1 bis 4, dadurch gekennzeichnet, daß als Agens zur Immunisierung an Albumin gebundenes Digoxinglutaryl-O-hydroxysuccinimid verwendet wird.

6. Verfahren nach einem der Ansprüche 1 bis 5, dadurch gekennzeichnet, daß die antikörperhaltige Globulinfraktion durch Ammonsulfatfällung aus dem Tierserum gewonnen wird.

7. Verfahren nach einem der Ansprüche 1 bis 6, dadurch gekennzeichnet, daß die Adsorption der Antikörper und die Elution der Begleitproteine mit einer auf pH 7 gepufferten Phosphat-/Natriumchloridlösung erfolgt.

8. Verfahren nach einem der Ansprüche 1 bis 7, dadurch gekennzeichnet, daß die durch Papain freigesetzten Fab-Fragmente mit einer wässrigen Salzsäure von der Säule eluiert werden.

9. Verfahren nach einem der Ansprüche 1 bis 8, dadurch gekennzeichnet, daß die von der Säule abgetrennten Fab-Fragmente durch Chromatographie an Gelen und/oder Ionenaustauschern weiter gereinigt werden.

10. Digitalisantikörper, erhältlich nach einem der Verfahren gemäß Anspruch 1 bis 9.

11. Verwendung von Digitalisantikörpern gemäß Anspruch 10, bei der Bekämpfung von Digitalisintoxikationen.

12. Verwendung von Digitalisantikörpern gemäß Anspruch 10, zur Herstellung von immunologischen Reagenzien zur Bestimmung von Digitalisglykosiden.

13. Reagenzien zur Bestimmung von Digitalisglykosiden, gekennzeichnet durch einen Gehalt von Digitalisantikörpern gemäß Anspruch 10.

**Claims**

1. Process for the preparation of digitalis antibodies, whereby suitable mammals are immunised with a digoxin bound to protein, the serum of the animals is recovered, the immunoglobulin-containing protein fraction is separated in per se known manner, the immunologically-active globulins are adsorbed on an immunologically-active column and separated from the other proteins, the antibodies are again eluted from the column, the Fab fractions are cleaved with papain and purified, characterised in that, as immunologically-active adsorbent, one uses an inorganic matrix of large surface area to which digitoxin aldehyde is covalently bound via a spacer not cleavable with papain and carries out the cleaving off of the Fab fraction from the antibodies on the matrix.

2. Process according to claim 1, characterised in that porous glass is used as inorganic carrier material.

3. Process according to one of claims 1 or 2, characterised in that triethoxysilylpropylamine is used as spacer.

4. Process according to one of claims 1 to 3, characterised in that digitoxin aldehyde obtained by oxidation of digitoxin with periodate is bound to the matrix as Schiff's base and the Schiff's base is reduced with sodium borohydride.

5. Process according to one of claims 1 to 4, characterised in that digoxinglutaryl-O-hydroxysuccinimide bound to albumin is used as agent for the immunisation.

6. Process according to one of claims 1 to 5, characterised in that the antibody-containing globulin fraction is obtained from the animal serum by ammonium sulphate precipitation.

7. Process according to one of claims 1 to 6, characterised in that the adsorption of the antibodies and the elution of the accompanying proteins takes place with a phosphate/sodium chloride solution buffered to pH 7.

8. Process according to one of claims 1 to 7, characterised in that the Fab fragments liberated by papain are eluted from the column with an aqueous hydrochloric acid.

9. Process according to one of claims 1 to 8, characterised in that the Fab fragments separated from the column are further purified by chromatography on gels and/or ion exchangers.

10. Digitalis antibodies obtainable according to a process according to claim 1 to 9.

**11.** Use of digitalis antibodies according to claim 10 in the combating of digitalis intoxications.

**12.** Use of digitalis antibodies according to claim 10 for the preparation of immunological reagents for the determination of digitalis glycosides.

**13.** Reagents for the determination of digitalis glycosides, characterised by a content of digitalis antibodies according to claim 10.

**Revendications**

**1.** Procédé pour la préparation d'anticorps dirigés contre la digitaline, dans lequel des mammifères appropriés sont immunisés au moyen d'une digoxine liée aux protéines, le sérum des animaux est recueilli, la fraction albuminique contenant l'immunoglobuline est séparée de manière connue en soi, les globulines immunologiquement actives sont adsorbées sur une colonne immunologiquement active et séparées des autres protéines, les anticorps sont réséparés de la colonne par élution, les fractions Fab sont séparées par l'intermédiaire de la papaïne et purifiées, procédé caractérisé en ce que, comme adsorbant immunologiquement actif, on utilise une matrice minérale à grande surface, sur laquelle est fixé le "spacer" digitoxinealdéhyde covalent, non séparable par la papaïne, et que la séparation de la fraction Fab d'avec l'anticorps est réalisée sur la matrice.

**2.** Procédé suivant la revendication 1, caractérisé en ce que comme matériau de support minéral, on utilise du verre poreux.

**3.** Procédé selon l'une des revendications 1 ou 2, caractérisé en ce que comme "spacer", on utilise la triéthoxysilylpropylamine.

**4.** Procédé selon l'une quelconque des revendications 1 à 3, caractérisé en ce que le digitoxinealdéhyde, obtenu par oxydation de la digitoxine à l'aide de periodate, est lié à la matrice en tant que base de Schiff et que cette base de Schiff est réduite à l'aide de borohydrure de sodium.

**5.** Procédé selon l'une quelconque des revendications 1 à 4, caractérisé en ce que comme agent d'immunisation, on utilise du digoxineglutaryl-O-hydroxysuccinimide lié à l'albumine.

**6.** Procédé selon l'une quelconque des revendications 1 à 5, caractérisé en ce que la fraction de globuline contenant les anticorps est obtenue à partir du sérum d'origine animale, par précipitation à l'aide de sulfate d'ammonium

**7.** Procédé selon l'une quelconque des revendications 1 à 6, caractérisé en ce que l'adsorption de l'anticorps et l'élution des protéines accompagnatrices sont réalisées à l'aide d'une solution phosphate-chlorure de sodium, tamponnée à pH7.

**8.** Procédé selon l'une quelconque des revendications 1 à 7, caractérisé en ce que les fragments Fab libérés au moyen de la papaïne sont élués de la colonne à l'aide d'une solution aqueuse d'acide chlorhydrique

**9.** Procédé selon l'une quelconque des revendications 1 à 8, caractérisé en ce que la purification ultérieure des fragments Fab séparés de la colonne est réalisée par chromatographie sur gel et/ou sur échangeurs d'ions.

**10.** Anticorps dirigé contre la digitaline, qui peut être obtenu selon l'un quelconque des procédés suivant les revendications 1 à 9.

**11.** Utilisation des anticorps dirigés contre la digitaline selon revendication 10 pour le traitement des intoxications de la digitaline.

**12.** Utilisation des anticorps dirigés contre la digitaline selons revendication 10 pour la préparation des reáctifs immunologiques pour la determination des glycosides de la digitaline.

**13.** Réactifs pour la determination des glycosides de la caractérisé digitaline par un contient des anticorps dirigés contre la digitaline selon revendication 10.